# EUROPEAN PATENT APPLICATION

(11) **EP 4 781 975 A1**
(43) Date of publication of application: **29.07.2026**
(21) Application number: 24868433.4
(22) Date of filing: 10.07.2024
(51) Int. Cl.: A61K 8/73, A61K 8/58, A61K 8/34, A61K 8/63, A61Q 19/00, A61L 26/00

(54) **SKIN TOPICAL COMPOSITION**

(30) Priority: 20.09.2023 KR 20230125539
(71) Applicant: Daebong LS Co., Ltd., Namdong-gu Incheon 21697 (KR)
(72) Inventor: PARK, Eun Ju, Hwaseong-si Gyeonggi-do 18425 (KR); LEE, Seon Mi, Incheon 21439 (KR); PARK, Jin Oh, Seoul 06276 (KR); LEE, Dong Su, Incheon 21931 (KR); BAEK, Seok Yun, Seongnam-si Gyeonggi-do 13611 (KR); LEE, Ji Won, Seoul 06276 (KR)
(74) Representative: Ter Meer Steinmeister & Partner
(86) International application number: PCT/KR2024/009834
(87) International publication number: WO 2025/063450

(57) **Abstract**

The present invention relates to a skin topical composition which is harmless to the human body and can fundamentally restore a skin barrier function. The composition comprises, as an active ingredient, a composite compound including: an Al-BA compound where alginate is conjugated with phenylboronic acid; and bacterial cellulose nanofibers ((+)BCNF) with positively charged surfaces. The skin topical composition of the present invention is harmless to the human body, can fundamentally restore the skin barrier function, and can be formulated into cosmetics, quasi-drugs, or pharmaceutical preparations.

## Description

### [Technical Field]

The skin plays an important role in protecting the human body from external physical and function.

### [Background Art]

The skin plays an important role in protecting the human body from external physical and chemical stimuli, while simultaneously retaining moisture and regulating body temperature. The skin is generally divided into three layers: the epidermis, dermis, and hypodermis. The epidermis is further divided, from the outermost layer inward, into the stratum corneum, stratum granulosum, stratum spinosum, and stratum basale.

Among these, the stratum corneum is located at the outermost layer of the epidermis and is composed of about 58% keratin proteins, about 38% natural moisturizing factors (NMF) that retain moisture, and about 11% lipids. The stratum corneum functions as a skin barrier. In other words, the stratum corneum contains protein-rich corneocytes and intercellular lipids including various lipids such as ceramides, free fatty acids, and cholesterol. The corneocytes and the intercorneocyte lipids form a multilamellar structure, thereby constituting an intact skin barrier. The primary functions of the skin barrier include preventing loss of body fluids and blocking invasion by toxins and pathogens.

However, the skin barrier is frequently damaged due to genetic or environmental factors. As a result, not only mechanical damage but also severe immune responses may occur due to the penetration of external irritants, toxins, or pathogens. In particular, with an increasing number of subjects engaging in tanning for beauty purposes, exposure to strong ultraviolet radiation has become more common. In addition, inflammatory responses caused by external irritants, such as particulate matter, as well as increased transepidermal water loss due to weakened barrier function, have contributed to an increasing incidence of skin damage-related diseases. Furthermore, when the skin barrier function is impaired, decreased moisture content in corneocytes causes rough skin, and in more severe cases, keratinization disorders in which corneocytes are shed may occur. Moreover, excessive immune responses triggered by external toxins or microorganisms penetrating through the weakened barrier may result in hyperkeratosis or related diseases (e.g., atopic dermatitis, psoriasis, etc.).

Conventionally, in order to address the problem of skin barrier damage, moisturizing factors (for example, hyaluronic acid, ceramides, and the like) have been supplemented to the skin. A representative example of related prior art is Korean Patent No. 2366360 (Patent Document 1; KR10-2366360 B1), the entire disclosure of which is incorporated herein by reference as prior art.

### [Prior Art Document]

### [Patent Document]

(Patent Document 1) KR 10-2366360B1 (2022.02.18.)

### [Disclosure]

### [Technical Problem]

As described above, conventional methods to solve skin barrier damage have primarily focused on supplementation of moisturizing factors. However, such methods have the drawback that the moisturizing effects decrease over time, causing the skin condition to revert to the original state thereof. Furthermore, these methods do not fundamentally restore the skin barrier and thus serve only as temporary measures.

Accordingly, an object of the present invention is to provide a cosmetic composition, a quasi-drug composition, or a pharmaceutical composition that is safe for topical application and is capable of restoring skin barrier function.

### [Technical Solution]

In accordance with the present invention, the above and other objects can be accomplished by the provision of
a skin topical composition containing, as active ingredients, (a) an Al-BA compound, in which phenylboronic acid is conjugated to alginate, and (b) bacterial cellulose nanofibers having a positively charged surface ((+)BCNF).

In the present invention, the composite of the Al-BA compound and (+)BCNF may be present in an amount of 0.1 to 10 wt% based on the total weight of the composition.

In the present invention, the Al-BA compound and bacterial cellulose nanofibers (BCNF) may be present in a weight ratio of 1: 0.01 to 1:0.5.

In the present invention, the composition may further contain one or more moisturizing agents or skin barrier ingredients selected from the group consisting of glycerin, hyaluronic acid, and cholesterol.

In the present invention, the composition may be a cosmetic composition for restoring skin barrier function and enhancing skin moisturization.

In the present invention, the composition may be a quasi-drug composition for protecting skin in areas where the skin barrier is damaged.

In the present invention, the quasi-drug composition may be provided as an adhesive wound dressing.

In the present invention, the composition may be a pharmaceutical composition for treating, preventing, or alleviating one or more diseases or symptoms selected from the group consisting of xerosis, pruritus, asteatotic eczema, cutaneous hyperkeratosis, keratinization of the heel and malleolar regions, senile xerosis, rough skin in adults, photodermatitis, atopic dermatitis, and psoriasis.

### [Advantageous effects]

The present invention provides a topical skin composition that restores skin barrier function. The composition may be formulated as a cosmetic composition, a quasi-drug composition, or a pharmaceutical composition.

### [Description of Drawings]

FIG. 1 is (a) a schematic diagram of alginate-boronic acid (Al-BA) conjugation, (b) FT-IR analysis to confirm synthesis of Al-BA, and (c) quantification of conjugated boronic acid;
FIG. 2 is (a) a schematic diagram of synthesis of positively charged bacterial cellulose nanofibers ((+)BCNF), (b) surface zeta potentials of natural cellulose depending on the amount of introduced glycidyltrimethylamine chloride (GTAC), and (c) a TEM image of (+)BCNF;
FIG. 3 is a graph showing results of recovery from skin barrier damage induced by chemical irritation;
FIG. 4 is a graph showing results of recovery from skin barrier damage induced by optical stimulation;
FIGS. 5 and 6 are graphs showing results of recovery from skin barrier damage induced by physical stimulation;
FIG. 7 is a graph showing the measured stratum corneum amount;
FIG. 8 is a graph showing the measured skin moisture content;
FIG. 9 is a graph showing the measured transepidermal water loss (TEWL);
FIG. 10 is a graph showing the measured skin roughness (skin texture);
FIG. 11 is a graph showing the results of visual evaluation of skin cracking;
FIG. 12 is a graph showing the measured skin moisture contents of the forearm depending on concentration and time;
FIG. 13 is a schematic diagram illustrating the overall schedule and scheme of the atopic dermatitis efficacy evaluation test;
FIGS. 14 to 17 are graphs showing test results for erythema/hemorrhage, scaling/dryness, edema, and excoriation/erosion symptoms at atopic lesion sites;
FIG. 18 is a graph showing the measured epidermal thickness of atopic dermatitis-induced skin; and
FIGS. 19 to 24 are images showing results of gross examination and histopathological examination.

### [Best Mode]

Terms used herein are defined as follows.

As used herein, the term "skin" refers to tissue covering the body surface of an animal, and encompasses, in the broadest sense, not only tissue covering the face or body but also the scalp and hair. Meanwhile, as used herein, the term "skin" may include not only living skin but also artificial skin or skin equivalents that mimic the condition of living skin.

As used herein, the term "skin barrier function" refers to all functions by which the skin, particularly the stratum corneum of the epidermis, acts as a barrier against the external environment, including prevention of transepidermal water loss and regulation of the penetration of external substances, but is not limited thereto.

As used herein, the term "skin barrier dysfunction" refers not only to a state in which the skin barrier function is deteriorated or damaged, but also to a state in which such deterioration or damage is likely to occur, and any state requiring prevention of such deterioration or damage, without limitation as to the type or severity of specific symptoms. More specifically, skin barrier dysfunction may include inflammatory responses caused by external stimuli such as ultraviolet radiation and particulate matter, as well as weakening of the epidermal layer due to aging, without being limited thereto.

As used herein, the term "amelioration" refers to any act of removing a state in which the skin barrier function is deteriorated or damaged, a state in which such deterioration or damage is likely to occur, or a state requiring prevention of such deterioration or damage, without limitation. More specifically, such amelioration ay include activation of autophagy in epidermal keratinocytes, which is reduced by external stimuli or aging, thereby promoting epidermal differentiation and turnover, and consequently contributing to restoration of the epidermal barrier function and prevention of aging, without being limited thereto.

As used herein, the term "treatment" refers to any act by which the symptoms of a disease are ameliorated or cured by administration of the composition according to the present invention.

As used herein, the term "prevention" refers to any act of suppressing or delaying the onset or progression of symptoms of a disease by administration of the composition according to the present invention.

As used herein, the term "alleviation" refers to any act by which symptoms of a disease are at least reduced or beneficially affected by administration of the pharmaceutical composition according to the present invention.

Hereinafter, the present invention will be described in detail.

The present invention provides a topical skin composition comprising, as active ingredients, an Al-BA compound in which phenylboronic acid is conjugated to alginate, and bacterial cellulose nanofibers having a positively charged surface ((+)BCNF).

The Al-BA compound is a complex compound in which phenylboronic acid is conjugated to alginate. The compound is capable of inducing adhesion to the skin through rapid sol-gel transition under physiological pH conditions.

The composite of the Al-BA compound and (+)BCNF may be present in an amount of 0.1 to 10 wt%, preferably 1 to 5 wt, based on the total weight of the composition. When the content is less than the above range, the intended effect on the skin barrier may be insufficient. When the content exceeds the above range, problems such as reduced formulation stability due to increased viscosity, reduced handling properties, and decreased dispersion uniformity of the preparation may occur.

The bacterial cellulose nanofibers may increase adhesion to the skin, and contribute to formation of a skin protective layer and recovery of damaged skin tissue. Due to the structure thereof similar to the extracellular matrix, the bacterial cellulose nanofibers may accelerate the proliferation rate of skin cells at damaged or wounded sites. In particular, the bacterial cellulose nanofibers are preferably positively charged on the surface thereof in order to enhance affinity with the A+l-BA compound and skin tissue, and to improve physical properties.

The content of the Al-BA complex compound may be 1 to 10 wt% based on the total weight of the composition.

The content of the bacterial cellulose nanofibers (BCNF) may be 1 wt% or less, preferably in the range of 0.01 to 0.5 wt%, based on the total weight of the composition.

In the present invention, it is preferable to consider the ratio of the Al-BA compound to the bacterial cellulose nanofibers (BCNF). Specifically, the weight ratio of Al-BA complex compound to BCNF may be within the range of 1: 0.01 to 0.5, preferably 1: 0.02 to 0.2, and more preferably 1: 0.03 to 0.16. Such weight ratios may provide more desirable physical properties for formation of a skin protective layer. Outside this range, differences in viscosity between the Al-BA compound and BCNF may result in non-uniform dispersion of polymers, which may impair adhesion to the skin and formation of the protective layer. For example, a composite compound containing about 3 wt% Al-BA complex compound and 0.25 wt% (+)BCNF may be used as an optimal concentration ratio.

The composition of the present invention may further contain, within a range that does not impair the effects of the present invention, moisturizing components such as glycerin and hyaluronic acid, as well as well-known skin barrier ingredients such as cholesterol, which functions as a mortar component in the so-called "brick and mortar model" of the skin barrier, without particular limitation. In addition, the composition may further contain conventional additives such as antioxidants, stabilizers, solubilizers, vitamins, pigments, fragrances, and carriers.

The skin topical composition of the present invention may be provided in any form of a cosmetic composition, a quasi-drug composition, or a pharmaceutical composition formulation that may contribute to restoration of the skin barrier function.

According to one embodiment, the skin topical composition may be prepared as a cosmetic composition. When used on a daily basis, the composition may provide effects of skin protection and recovery of damaged skin, while continuously improving the skin barrier function without causing skin trouble, thereby contributing to overall improvement of skin condition. The cosmetic composition according to one embodiment of the present invention is not limited to any particular formulation and may be prepared in any form commonly used in the art, including, for example, a solution, gel, solid, anhydrous paste, emulsion, suspension, microemulsion, microcapsule, fine granule, liposome, nonionic vesicle dispersion, serum, cream, toner, lotion, powder, spray, paste, pack, cleanser, soap, oil, wax, concealer stick, or aerosol formulation, without limitation. More specifically, the composition may be applied to functional cosmetic products such as toners, nourishing creams, moisturizing creams, essences, eye creams, lotions, packs, and other basic cosmetic products.

The content of the extract contained as an active ingredient in the cosmetic composition according to the present invention may be appropriately determined in consideration of various factors such as the intended use, duration of use, formulation type, route of application, and skin condition of the subject. For example, the extract may be present in an amount of 0.0001 to 10 wt%, for example 0.001 to 5 wt%, based on the total weight of the cosmetic composition.

According to another embodiment, the present invention may be provided as a quasi-drug composition for improving skin barrier function. As used herein, the term "quasi-drug" refers to a product used to diagnose, treat, ameliorate, alleviate, or prevent diseases in humans or animals, but having milder effects than pharmaceutical products. For example, according to applicable regulations, quasi-drugs may include products used for treatment or prevention of diseases in humans or animals, or products having mild or indirect effects on the human body, excluding pharmaceutical products.

Similar to the cosmetic composition described above, the quasi-drug composition may be used to enhance skin moisturization and restore damaged skin barrier function. The quasi-drug composition may also be used to protect damaged areas of the skin barrier, and may further be used as a medical device such as an adhesive (transparent) wound dressing to cover and protect skin wounds caused by various factors.

Examples of the quasi-drug composition of the present invention may include disinfectants, cleansing agents, shower foams, ointments, wet tissues, and coating agents. Preferably, the composition may be prepared as a semi-solid formulation such as a topical ointment or lotion, but is not limited thereto. Methods of formulating the quasi-drug, and dosage, method of use, and ingredients thereof may be appropriately selected from techniques commonly known in the art.

The content of the extract present as an active ingredient in the quasi-drug composition according to the present invention may be appropriately determined in consideration of various factors such as intended use, duration of use, formulation type, route of application, and skin condition of the subject. For example, the active ingredient may be present in an amount of 0.0001 to 10 wt %, for example 0.001 to 5 wt %, based on the total weight of the quasi-drug composition.

According to another embodiment, the present invention may be used as a pharmaceutical composition for treating, preventing, or alleviating diseases associated with skin barrier damage. The diseases associated with skin barrier damage may include various infectious and inflammatory diseases that damage the skin barrier or are caused by skin barrier impairment. Specific examples thereof include, but are not limited to, xerosis, pruritus, asteatotic eczema, cutaneous hyperkeratosis, keratosis of the heel and malleolar regions, senile xerosis, rough skin in adults, photodermatitis, atopic dermatitis, and psoriasis.

The pharmaceutical composition of the present invention may be prepared in any formulation commonly used in the art. For example, the composition may be prepared as a solution, gel, solid, anhydrous paste, emulsion, suspension, microemulsion, microcapsule, fine granule, liposome, nonionic vesicle dispersion, serum, cream, toner, lotion, powder, spray, paste, pack, cleanser, soap, oil, wax, concealer stick, or aerosol formulation.

Hereinafter, the present invention will be described in more detail with reference to the following examples and experimental examples. However, these examples are provided for illustrative purposes only and are not intended to limit the scope of the present invention in any manner.

### Example

### Preparation of Al-BA/(+)BCNF composite compound

**Synthesis of Al-BA Compound**: first, an alginate polymer was dispersed in MES buffer (0.1 M, pH 5.0) at room temperature. A mixed solution of ethyl-3-(3-dimethylaminopropyl)carbodiimide (EDC) and N-hydroxysuccinimide (NHS) was slowly added to the alginate dispersion under stirring. Then, a solution of 3-aminophenylboronic acid was slowly added thereto and the reaction mixture was stirred for 24 hours. After completion of the reaction, residual reactants were removed through dialysis to obtain alginate-boronic acid (Al-BA). The prepared Al-BA was freeze-dried and stored.

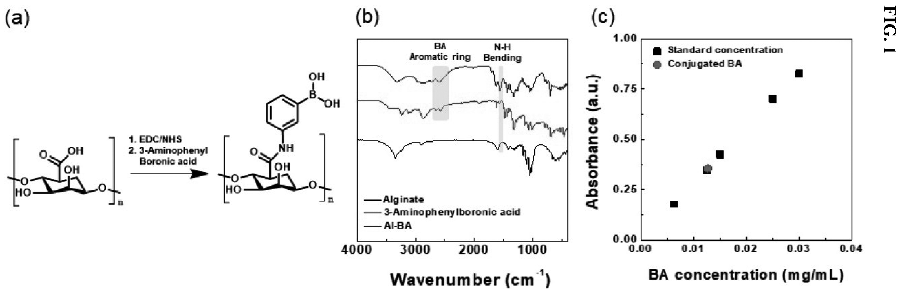

**Identification of Al-BA compound**: FT-IR analysis was performed before and after addiiton of 3-aminophenylboronic acid into the alginate polymer. Similar characteristic peaks corresponding to 3-aminophenylboronic acid were observed in the regions of 3,000-2,800 cm⁻¹ and 1,650-1,580 cm⁻¹, which indicates successful synthesis. Quantification of the added 3-aminophenylboronic acid (BA) was further performed by UV-Vis analysis. The results are shown in FIG. 1.

**Synthesis of BCNF:** Natural cellulose raw material was dispersed in 0.1 M NaOH solution and a predetermined amount of glycidyltrimethylammonium chloride (GTAC) was added thereto. The mixture was stirred at 60°C for 2 hours. Then, residual materials were removed by centrifugation and the reaction product was re-dispersed in a 0.01 M NaOH solution. NH₄OH was then added to the cellulose re-dispersion, followed by stirring at 60°C for 2 hours and centrifugation, to obtain positively charged natural cellulose nanofibers ((+)BCNF).

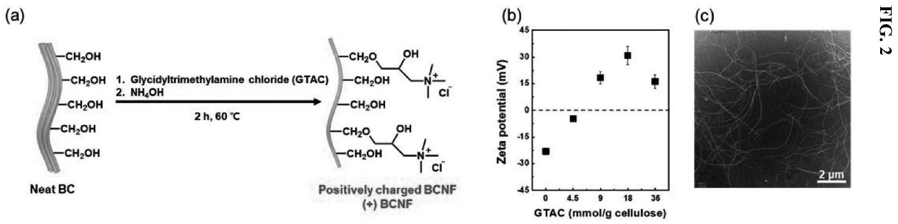

**Identification of BCNF:** Surface potential analysis was conducted depending on the amount of glycidyltrimethylammonium chloride (GTAC) introduced onto the surface of the natural cellulose nanofibers. As the amount of introduced GTAC increased, the surface potential shifted toward the positive range. The nanofiber structure of the prepared (+)BCNF was visualized by transmission electron microscopy (TEM). The results are shown in FIG. 2.

**Preparation of Al-BA/(+)BCNF compound:** the prepared Al-BA and the obtained (+)BCNF were added to water at concentrations of 3 wt% and 0.25 wt%, respectively, and mixed thoroughly to prepare a hydrogel-type composite of Al-BA/(+)BCNF.

Using the Al-BA/(+)BCNF composite prepared as described above, as an active ingredient, compositions of Example 1, Example 2, and Comparative Example were prepared as shown in Table 1 (unit: wt%).

**[Table 1]**

| Name of ingredient | Comparative Example | Example 1 | Example 2 |
|---|---|---|---|
| Purified water | TO.100 | TO.100 | TO.100 |
| Glycerin | 5.0000 | 5.0000 | 5.0000 |
| Sodium polyacrylate | 0.3000 | 0.3000 | 0.3000 |
| Xanthan gum | 0.0500 | 0.0500 | 0.0500 |
| Cetyl ethylhexanoate | 8.0000 | 8.0000 | 8.0000 |
| Hydrogenated polydecene | 4.0000 | 4.0000 | 4.0000 |
| Glyceryl stearate | 3.0400 | 3.0400 | 3.0400 |
| Cetearyl alcohol | 3.0000 | 3.0000 | 3.0000 |
| Cholesterol | 2.8680 | 2.8680 | 2.8680 |
| Dimethicone | 2.0000 | 2.0000 | 2.0000 |
| Hydroxypropyl bispalmitamide MEA | 2.0000 | 2.0000 | 2.0000 |
| PEG stearate | 0.9600 | 0.9600 | 0.9600 |
| Glyceryl caprylate | 0.5000 | 0.5000 | 0.5000 |
| Candelilla wax | 0.5000 | 0.5000 | 0.5000 |
| Palmitic acid | 0.0546 | 0.0546 | 0.0546 |
| Stearic acid | 0.0442 | 0.0442 | 0.0442 |
| Lauric acid | 0.0006 | 0.0006 | 0.0006 |
| Myristic acid | 0.0006 | 0.0006 | 0.0006 |
| Purified water | 2.0000 | 2.0000 | 2.0000 |
| Arginine | 0.2000 | 0.2000 | 0.2000 |
| Al-BA/(+)BCNF | - | 0.5000 | 1.0000 |
| 1,2-hexanediol | 1.0000 | 1.0000 | 1.0000 |

### Safety and efficacy evaluation

In order to evaluate the efficacy and safety of a formulation containing Al-BA/(+)BCNF, and to determine various effects such as restoration of skin barrier function and enhancement of skin moisturization, clinical tests for various parameters were conducted as described below.

### 1. Information on subjects and test product

A total of 11 subjects participated in the clinical test, and the general information thereof is shown in Table 2 below.

**[Table 2]**

| **Subject identification code** | **Name** | **Date of birth** | **Age (in years)** | **Skin type** | **Gender** |
|---|---|---|---|---|---|
| 23118-PT1-01 | KKM | 1982-04-08 | 40 | Normal | Female |
| 23118-PT1-02 | JEJ | 2001-01-17 | 22 | Normal-to-dry | Female |
| 23118-PT1-03 | KHJ | 1992-01-27 | 30 | Normal | Female |
| 23118-PT1-04 | HJS | 1969-01-25 | 53 | Dry | Female |
| 23118-PT1-05 | LSM | 1972-02-01 | 50 | Normal | Female |
| 23118-PT1-06 | KYN | 1988-01-03 | 35 | Normal | Female |
| 23118-PT1-07 | JYH | 1986-04-23 | 36 | Dry | Female |
| 23118-PT1-08 | CYL | 1977-07-28 | 45 | Normal-to-dry | Female |
| 23118-PT1-09 | KSM | 1969-02-15 | 54 | Dry | Female |
| 23118-PT1-10 | KKY | 1972-09-10 | 50 | Dry | Female |
| 23118-PT1-11 | KMS | 1992-08-04 | 30 | Dry | Female |

Meanwhile, the test product used herein was Example 2 prepared as described above and the control product used herein was Comparative Example prepared as described above.

### 2. Evaluation of recovery from skin barrier damage induced by chemical irritation

### [Test Method]

In order to induce skin barrier damage by chemical irritation, a test area was marked on the left forearm of each subject where no pigmentation or visible skin damage was present. A 2% sodium lauryl sulfate (SLS) solution was applied under occlusion for 24 hours using an IQ Ultimate chamber (Chemotechnique Diagnostics, Sweden) to induce skin damage.

Recovery from skin barrier damage induced by chemical irritation was evaluated using a Vapometer (Delfin Technologies Ltd., Finland). Measurements were taken at the same site on the left forearm before SLS application, 24 hours after SLS application, and after 2 weeks of use of the test product. The measurement unit was g/m²h. A lower measured value indicates an improved effect in recovery from skin barrier damage induced by chemical irritation.

### [Test Results]

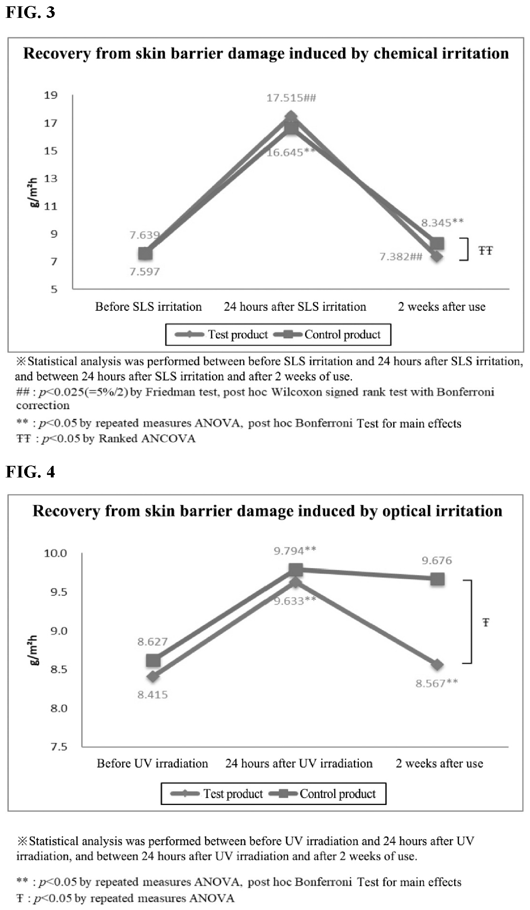

The results of the evaluation of recovery from skin barrier damage induced by chemical irritation (unit: g/m²·h) are shown in Table 3 and FIG. 3.

**[Table 3]**

| **Subject identification code** | **Test product** | | |
|---|---|---|---|
| | **Before SLS irritation** | **24 hours after SLS irritation** | **2 weeks after use** |
| 23118-PT1-01 | 9.033 | 38.000 | 8.833 |
| 23118-PT1-02 | 6.233 | 12.767 | 6.767 |
| 23118-PT1-03 | 6.867 | 14.600 | 8.300 |
| 23118-PT1-04 | 8.633 | 14.533 | 7.833 |
| 23118-PT1-05 | 5.400 | 15.500 | 4.833 |
| 23118-PT1-06 | 7.533 | 18.200 | 7.800 |
| 23118-PT1-07 | 6.300 | 13.400 | 6.800 |
| 23118-PT1-08 | 8.033 | 15.867 | 7.200 |
| 23118-PT1-09 | 10.167 | 19.633 | 8.167 |
| 23118-PT1-10 | 10.500 | 19.867 | 9.400 |
| 23118-PT1-11 | 5.333 | 10.300 | 5.267 |
| Mean | 7.639 | 17.515 | 7.382 |

| **Subject identification code** | **Control product** | | |
|---|---|---|---|
| | **Before SLS irritation** | **24 hours after SLS irritation** | **2 weeks after use** |
| 23118-PT1-01 | 9.400 | 28.767 | 10.167 |
| 23118-PT1-02 | 5.967 | 13.700 | 8.200 |
| 23118-PT1-03 | 6.733 | 14.433 | 9.167 |
| 23118-PT1-04 | 8.600 | 13.467 | 9.800 |
| 23118-PT1-05 | 5.567 | 15.433 | 5.933 |
| 23118-PT1-06 | 7.433 | 18.500 | 8.233 |
| 23118-PT1-07 | 6.567 | 13.433 | 7.000 |
| 23118-PT1-08 | 8.100 | 15.900 | 8.000 |
| 23118-PT1-09 | 9.500 | 18.767 | 8.333 |
| 23118-PT1-10 | 10.467 | 20.467 | 9.800 |
| 23118-PT1-11 | 5.233 | 10.233 | 7.167 |
| Mean | 7.597 | 16.645 | 8.345 |

- Test product application site: Compared with before SLS irritation, the measured value statistically significantly increased 24 hours after SLS irritation. Furthermore, compared with 24 hours after SLS irritation, a statistically significant improvement was observed after 2 weeks of product use.
- Control product application site: Compared with before SLS irritation, the measured value statistically significantly increased 24 hours after SLS irritation. In addition, compared with 24 hours after SLS irritation, a statistically significant improvement was observed after 2 weeks of use.
- Between-group comparison: No statistically significant difference was observed between the groups at 24 hours after SLS irritation compared with before SLS irritation. However, a statistically significant difference between the groups was observed after 2 weeks of use compared with 24 hours after SLS irritation.

### 3. Evaluation of recovery from skin barrier damage induced by optical irritation

### [Test Method]

To induce skin barrier damage by optical irritation, ultraviolet (UV) irradiation was performed using a Multiport Solar Simulator, 601-300W (Solar Light, U.S.A.). A test area was marked on the left forearm of each subject where no pigmentation or visible skin damage was present. UV irradiation at the same intensity (650 µW/cm²) was applied for 1 minute and 30 seconds (±10 seconds).

Recovery of skin barrier damage induced by optical irritation was measured on the same right forearm site using a Vapometer (Delfin Technologies Ltd., Finland) at three time points: before UV irradiation, 24 hours after UV irradiation, and after 2 weeks of use of the test product. The measurement unit was g/m²h. A lower measured value indicates an improved effect in recovery from skin barrier damage induced by optical irritation.

### [Test Results]

The results of measurement of recovery from skin barrier damage induced by optical irritation (unit: g/m²h) are shown in Table 4 and FIG. 4 below.

**[Table 4]**

| **Subject identification code** | **Test product** | | |
|---|---|---|---|
| | **Before UV irradiation** | **24 hours after UV irradiation** | **2 weeks after use** |
| 23118-PT1-01 | 8.867 | 10.367 | 8.233 |
| 23118-PT1-02 | 8.300 | 9.067 | 8.067 |
| 23118-PT1-03 | 9.100 | 10.633 | 9.500 |
| 23118-PT1-04 | 8.500 | 9.100 | 8.533 |
| 23118-PT1-05 | 6.400 | 8.167 | 5.733 |
| 23118-PT1-06 | 8.700 | 9.333 | 8.900 |
| 23118-PT1-07 | 6.667 | 8.300 | 8.000 |
| 23118-PT1-08 | 8.600 | 9.200 | 9.067 |
| 23118-PT1-09 | 10.500 | 12.033 | 10.500 |
| 23118-PT1-10 | 11.433 | 12.600 | 11.067 |
| 23118-PT1-11 | 5.500 | 7.167 | 6.633 |
| Mean | 8.415 | 9.633 | 8.567 |

| **Subject identification code** | **Control product** | | |
|---|---|---|---|
| | **Before UV irradiation** | **24 hours after UV irradiation** | **2 weeks after use** |
| 23118-PT1-01 | 8.667 | 10.100 | 11.500 |
| 23118-PT1-02 | 8.500 | 9.400 | 8.867 |
| 23118-PT1-03 | 8.733 | 10.200 | 11.200 |
| 23118-PT1-04 | 8.233 | 9.000 | 8.700 |
| 23118-PT1-05 | 6.500 | 8.100 | 7.533 |
| 23118-PT1-06 | 8.167 | 9.567 | 9.033 |
| 23118-PT1-07 | 7.733 | 9.100 | 8.700 |
| 23118-PT1-08 | 8.300 | 9.667 | 9.300 |
| 23118-PT1-09 | 11.200 | 12.333 | 11.833 |
| 23118-PT1-10 | 12.067 | 13.233 | 12.267 |
| 23118-PT1-11 | 6.800 | 7.033 | 7.500 |
| Mean | 8.627 | 9.794 | 9.676 |

- Test product application site: Compared with before UV irradiation, the measured value statistically significantly increased 24 hours after UV irradiation. Furthermore, compared with 24 hours after UV irradiation, a statistically significant improvement was observed after 2 weeks of use.
- Control product application site: Compared with before UV irradiation, the measured value statistically significantly increased 24 hours after UV irradiation.
- Between-group comparison:

No statistically significant difference was observed between the groups at 24 hours after UV irradiation compared with before UV irradiation. However, a statistically significant difference between the groups was observed after 2 weeks of use compared with 24 hours after UV irradiation.

### 4. Evaluation of recovery from skin barrier damage induced by physical irritation

### [Test Method]

To induce skin barrier damage by physical irritation, a test area was marked on the left forearm of each subject where no pigmentation or visible skin damage was present. Skin damage was induced by repeated application and removal of adhesive film (D-Squame^{®}; Cuderm, USA), a process commonly referred to as a "tape stripping" (TS).

Recovery of skin barrier damage induced by physical irritation was measured using a Vapometer (Delfin Technologies Ltd., Finland). Measurements were performed at three time points on the same left forearm site: before tape stripping (TS), after tape stripping, and immediately after application of the test product. The measurement unit was g/m²h. A lower measured value indicates an improved effect in recovery from skin barrier damage induced by oprical irritation.

In addition, measurements were also performed using Antera 3D CS (Miravex Ltd., Ireland) on the same left forearm site at three time points: before tape stripping (TS), after TS, and immediately after application of the test product. The obtained images (color mode) were analyzed by designating specific regions of interest (ROIs) within each segmented area. The evaluated parameter was a*, which represents skin redness. A decrease in a* immediately after application compared with after TS indicates an improved effect on recovery from skin barrier damage induced by physical irritation.

### [Test Results]

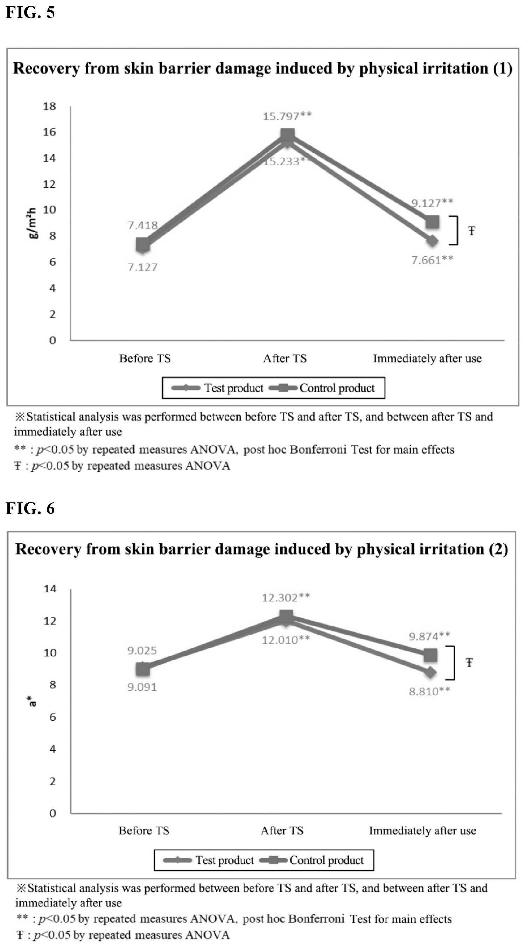

The results of measurement of recovery from skin barrier damage induced by physical irritation are shown in Table 5 (unit: g/m²h), Table 6 (unit: a*), and FIGS. 5 and 6 below.

**[Table 5]**

| **Subject identification code** | **Test product** | | |
|---|---|---|---|
| | **Before TS** | **After TS** | **Immediately after use** |
| 23118-PT1-01 | 8.000 | 16.433 | 7.300 |
| 23118-PT1-02 | 6.800 | 15.633 | 8.133 |
| 23118-PT1-03 | 7.633 | 16.200 | 7.267 |
| 23118-PT1-04 | 6.833 | 15.600 | 6.567 |
| 23118-PT1-05 | 6.733 | 14.367 | 8.933 |
| 23118-PT1-06 | 8.700 | 16.400 | 8.167 |
| 23118-PT1-07 | 6.667 | 12.933 | 7.067 |
| 23118-PT1-08 | 6.533 | 13.100 | 5.833 |
| 23118-PT1-09 | 5.900 | 14.933 | 7.400 |
| 23118-PT1-10 | 9.733 | 20.433 | 11.000 |
| 23118-PT1-11 | 4.867 | 11.533 | 6.600 |
| Mean | 7.127 | 15.233 | 7.661 |

| **Subject identification code** | **Control product** | | |
|---|---|---|---|
| | **Before TS** | **After TS** | **Immediately after use** |
| 23118-PT1-01 | 8.867 | 16.133 | 9.367 |
| 23118-PT1-02 | 7.133 | 14.033 | 8.400 |
| 23118-PT1-03 | 9.300 | 18.267 | 10.067 |
| 23118-PT1-04 | 6.500 | 15.900 | 7.067 |
| 23118-PT1-05 | 8.733 | 19.067 | 14.167 |
| 23118-PT1-06 | 8.033 | 16.833 | 9.533 |
| 23118-PT1-07 | 6.867 | 12.067 | 8.233 |
| 23118-PT1-08 | 6.867 | 14.900 | 7.267 |
| 23118-PT1-09 | 5.533 | 14.967 | 7.700 |
| 23118-PT1-10 | 9.133 | 19.400 | 11.933 |
| 23118-PT1-11 | 4.633 | 12.200 | 6.667 |
| Mean | 7.418 | 15.797 | 9.127 |

**[Table 6]**

| **Subject identification code** | **Test product** | | |
|---|---|---|---|
| | **Before TS** | **After TS** | **Immediately after use** |
| 23118-PT1-01 | 7.557 | 11.171 | 7.740 |
| 23118-PT1-02 | 9.022 | 13.446 | 8.955 |
| 23118-PT1-03 | 6.578 | 8.081 | 6.182 |
| 23118-PT1-04 | 8.219 | 9.300 | 6.753 |
| 23118-PT1-05 | 7.983 | 12.131 | 8.183 |
| 23118-PT1-06 | 8.465 | 9.235 | 7.827 |
| 23118-PT1-07 | 11.371 | 14.082 | 11.570 |
| 23118-PT1-08 | 9.622 | 12.672 | 9.330 |
| 23118-PT1-09 | 10.799 | 14.153 | 9.879 |
| 23118-PT1-10 | 9.235 | 12.782 | 9.184 |
| 23118-PT1-11 | 11.152 | 15.053 | 11.312 |
| Mean | 9.091 | 12.010 | 8.810 |

| **Subject identification code** | **Control product** | | |
|---|---|---|---|
| | **Before TS** | **After TS** | **Immediately after use** |
| 23118-PT1-01 | 7.230 | 10.434 | 8.680 |
| 23118-PT1-02 | 8.465 | 12.340 | 8.716 |
| 23118-PT1-03 | 5.863 | 8.088 | 6.650 |
| 23118-PT1-04 | 7.562 | 9.129 | 8.369 |
| 23118-PT1-05 | 7.847 | 11.806 | 9.146 |
| 23118-PT1-06 | 8.173 | 10.380 | 8.754 |
| 23118-PT1-07 | 12.033 | 15.228 | 13.251 |
| 23118-PT1-08 | 9.121 | 13.435 | 10.679 |
| 23118-PT1-09 | 10.583 | 14.922 | 11.088 |
| 23118-PT1-10 | 10.464 | 14.249 | 11.133 |
| 23118-PT1-11 | 11.929 | 15.307 | 12.151 |
| Mean | 9.025 | 12.302 | 9.874 |

- Test product application site: Compared with before tape stripping (TS), the measured value statistically significantly increased after TS. Furthermore, compared with after TS, a statistically significant improvement was observed immediately after application.
- Control product application site: Compared with before TS, the measured value statistically significantly increased after TS. In addition, compared with after TS, a statistically significant improvement was observed immediately after application.
- Between-group comparison: No statistically significant difference was observed between the groups after TS compared with before TS. However, a statistically significant difference between the groups was observed immediately after application compared with after TS.

### 5. Measurement of stratum corneum amount

### [Test Method]

Stratum corneum content was measured using a special adhesive film (D-Squame^{®}; Cuderm, USA). Stratum corneum samples were collected from the same bilateral heel areas of the subjects at three time points: before product use, immediately after use, and after 2 weeks of use. The collected samples were imaged using a Visioscan^{®} VC20 (Courage + Khazaka electronic GmbH, Germany). The evaluated parameter was D.I. (desquamation index, %) and a lower value indicates an improved effect on stratum corneum amount.

### [Test Results]

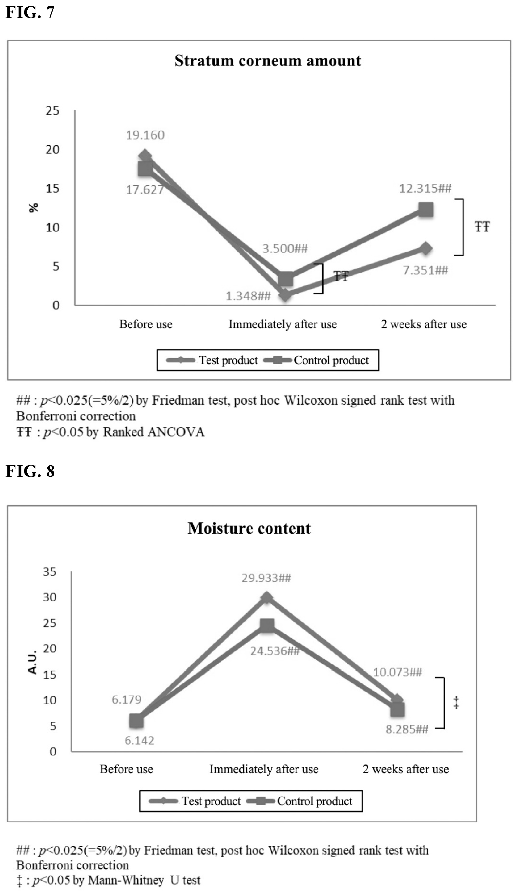

The results of measurement of stratum corneum amount are shown in Table 7 (unit: %) and FIG. 7 below.

**[Table 7]**

| **Subject identification code** | **Test product** | | |
|---|---|---|---|
| | **Before use** | **Immediately after use** | **2 weeks after use** |
| 23118-PT1-01 | 27.61 | 1.82 | 14.10 |
| 23118-PT1-02 | 32.80 | 1.48 | 3.57 |
| 23118-PT1-03 | 13.85 | 2.15 | 3.98 |
| 23118-PT1-04 | 22.48 | 2.73 | 9.22 |
| 23118-PT1-05 | 2.75 | 0.61 | 2.05 |
| 23118-PT1-06 | 21.46 | 0.87 | 3.53 |
| 23118-PT1-07 | 15.80 | 1.09 | 4.50 |
| 23118-PT1-08 | 6.05 | 1.11 | 1.95 |
| 23118-PT1-09 | 5.00 | 0.33 | 1.86 |
| 23118-PT1-10 | 27.79 | 1.00 | 16.72 |
| 23118-PT1-11 | 35.17 | 1.64 | 19.38 |
| Mean | 19.160 | 1.348 | 7.351 |

| **Subject identification code** | **Control product** | | |
|---|---|---|---|
| | **Before use** | **Immediately after use** | **2 weeks after use** |
| 23118-PT1-01 | 23.77 | 3.51 | 18.60 |
| 23118-PT1-02 | 28.39 | 1.62 | 8.46 |
| 23118-PT1-03 | 21.68 | 4.17 | 18.13 |
| 23118-PT1-04 | 18.43 | 5.74 | 13.45 |
| 23118-PT1-05 | 12.64 | 0.71 | 6.88 |
| 23118-PT1-06 | 9.87 | 4.94 | 5.70 |
| 23118-PT1-07 | 15.59 | 10.83 | 15.24 |
| 23118-PT1-08 | 2.89 | 0.80 | 2.62 |
| 23118-PT1-09 | 2.96 | 0.85 | 2.38 |
| 23118-PT1-10 | 22.59 | 1.95 | 21.40 |
| 23118-PT1-11 | 35.09 | 3.38 | 22.60 |
| Mean | 17.627 | 3.500 | 12.315 |

- Test product application site: Compared with before use, statistically significant improvements were observed immediately after use and after 2 weeks of use.
- Control product application site: Compared with before use, statistically significant improvements were observed immediately after use and after 2 weeks of use.
- Between-group comparison: Compared with before use, statistically significant differences between the groups were observed immediately after use and after 2 weeks of use.

### 6. Measurement of skin moisture content

### [Test Method]

Skin moisture content was measured using a Corneometer^{®} CM825 (Courage + Khazaka electronic GmbH, Germany) on the same bilateral heel areas of the subjects at three time points: before product use, immediately after use, and after 2 weeks of use. The evaluated parameter was an arbitrary unit (A.U.). A higher arbitrary unit value indicates an improved effect on the skin moisture content.

### [Test results]

The results of measurement of the skin moisture content are shown in Table 8 (unit: A.U.) and FIG. 8 below.

**[Table 8]**

| **Subject identification code** | **Test product** | | |
|---|---|---|---|
| | **Before use** | **Immediately after use** | **2 weeks after use** |
| 23118-PT1-01 | 3.400 | 35.433 | 5.600 |
| 23118-PT1-02 | 14.267 | 30.467 | 15.533 |
| 23118-PT1-03 | 5.567 | 31.400 | 8.400 |
| 23118-PT1-04 | 7.200 | 35.567 | 9.233 |
| 23118-PT1-05 | 2.500 | 35.267 | 4.400 |
| 23118-PT1-06 | 4.433 | 38.600 | 13.533 |
| 23118-PT1-07 | 8.100 | 20.600 | 12.033 |
| 23118-PT1-08 | 4.400 | 28.500 | 7.467 |
| 23118-PT1-09 | 6.633 | 25.467 | 15.467 |
| 23118-PT1-10 | 4.500 | 25.567 | 10.233 |
| 23118-PT1-11 | 6.567 | 22.400 | 8.900 |
| Mean | 6.142 | 29.933 | 10.073 |

| **Subject identification code** | **Control product** | | |
|---|---|---|---|
| | **Before use** | **Immediately after use** | **2 weeks after use** |
| 23118-PT1-01 | 4.167 | 33.300 | 5.100 |
| 23118-PT1-02 | 14.500 | 28.500 | 15.200 |
| 23118-PT1-03 | 5.467 | 28.733 | 6.733 |
| 23118-PT1-04 | 7.533 | 35.467 | 8.100 |
| 23118-PT1-05 | 2.467 | 34.533 | 2.800 |
| 23118-PT1-06 | 5.133 | 25.700 | 10.567 |
| 23118-PT1-07 | 7.700 | 15.600 | 7.567 |
| 23118-PT1-08 | 5.033 | 12.400 | 6.433 |
| 23118-PT1-09 | 5.533 | 22.367 | 12.567 |
| 23118-PT1-10 | 4.700 | 19.000 | 9.467 |
| 23118-PT1-11 | 5.733 | 14.300 | 6.600 |
| Mean | 6.179 | 24.536 | 8.285 |

- Test product application site: Compared with before use, statistically significant improvements were observed immediately after use and after 2 weeks of use.
- Control product application site: Compared with before use, statistically significant improvements were observed immediately after use and after 2 weeks of use.
- Between-group comparison: Compared with before use, statistically significant differences between the groups were observed immediately after use and after 2 weeks of use.

### 7. Measurement of transepidermal water loss (TEWL)

### [Test Method]

Transepidermal water loss (TEWL) was measured using a Vapometer^{®} (Delfin, Finland) on the same bilateral heel areas of the subjects before product use, immediately after use, and after 2 weeks of use. The parameter was water loss (g/m²h), and a lower value indicates improved transepidermal water loss.

### [Test Method]

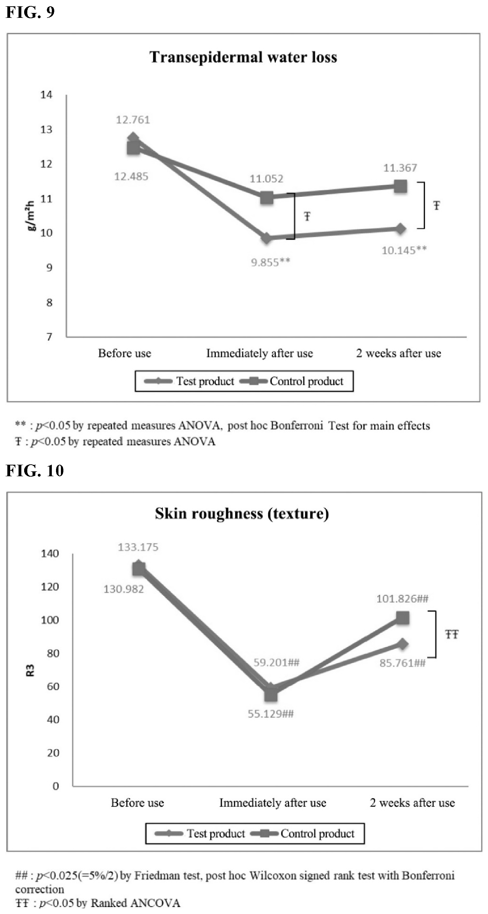

The results of measurement of water loss are shown in Table 9 (unit: g/m²h) and FIG. 9 below.

**[Table 9]**

| **Subject identification code** | **Test product** | | |
|---|---|---|---|
| | **Before use** | **Immediately after use** | **2 weeks after use** |
| 23118-PT1-01 | 9.700 | 8.133 | 8.467 |
| 23118-PT1-02 | 20.333 | 12.600 | 13.400 |
| 23118-PT1-03 | 16.500 | 15.467 | 10.567 |
| 23118-PT1-04 | 14.400 | 10.433 | 13.633 |
| 23118-PT1-05 | 12.633 | 6.667 | 9.400 |
| 23118-PT1-06 | 7.200 | 6.200 | 6.000 |
| 23118-PT1-07 | 7.000 | 5.000 | 6.133 |
| 23118-PT1-08 | 15.333 | 12.667 | 13.267 |
| 23118-PT1-09 | 9.567 | 9.233 | 8.133 |
| 23118-PT1-10 | 14.400 | 12.467 | 14.133 |
| 23118-PT1-11 | 13.300 | 9.533 | 8.467 |
| Mean | 12.761 | 9.855 | 10.145 |

| **Subject identification code** | **Control product** | | |
|---|---|---|---|
| | **Before use** | **Immediately after use** | **2 weeks after use** |
| 23118-PT1-01 | 10.400 | 9.533 | 10.067 |
| 23118-PT1-02 | 21.500 | 16.200 | 14.333 |
| 23118-PT1-03 | 15.533 | 14.933 | 14.900 |
| 23118-PT1-04 | 15.267 | 11.800 | 14.533 |
| 23118-PT1-05 | 11.533 | 9.367 | 10.367 |
| 23118-PT1-06 | 7.033 | 6.933 | 6.733 |
| 23118-PT1-07 | 6.500 | 6.467 | 6.433 |
| 23118-PT1-08 | 13.267 | 12.433 | 13.133 |
| 23118-PT1-09 | 10.233 | 10.167 | 9.533 |
| 23118-PT1-10 | 13.467 | 13.233 | 13.400 |
| 23118-PT1-11 | 12.600 | 10.500 | 11.600 |
| Mean | 12.485 | 11.052 | 11.367 |

- Test product application site: Compared with before use, statistically significant improvements were observed immediately after use and after 2 weeks of use.
- Control product application site: Compared with before use, no statistically significant improvements were observed.
- Between-group comparison: Compared with before use, statistically significant differences between the groups were observed immediately after use and after 2 weeks of use.

### 8. Measurement of skin roughness

### [Test Method]

Skin roughness (skin texture) was measured using a Visioscan^{®} VC98 (Courage + Khazaka electronic GmbH, Germany) on the same bilateral heel areas of the subjects at three time points: before product use, immediately after use, and after 2 weeks of use. The evaluation parameter was R3 (Roughness). A lower R3 value indicates an improved skin roughness (skin texture).

### [Test Results]

The results of the measurement of skin roughness (skin texture) are shown in Table 10 (unit: R3) and FIG. 10 below.

**[Table 10]**

| **Subject identification code** | **Test product** | | |
|---|---|---|---|
| | **Before use** | **Immediately after use** | **2 weeks after use** |
| 23118-PT1-01 | 132.755 | 64.465 | 80.925 |
| 23118-PT1-02 | 119.005 | 45.070 | 92.010 |
| 23118-PT1-03 | 146.275 | 86.525 | 55.290 |
| 23118-PT1-04 | 127.615 | 51.480 | 75.150 |
| 23118-PT1-05 | 130.995 | 53.725 | 81.230 |
| 23118-PT1-06 | 127.980 | 57.660 | 93.005 |
| 23118-PT1-07 | 144.145 | 53.360 | 92.325 |
| 23118-PT1-08 | 134.605 | 60.230 | 109.260 |
| 23118-PT1-09 | 150.690 | 86.675 | 80.710 |
| 23118-PT1-10 | 132.795 | 45.570 | 91.365 |
| 23118-PT1-11 | 118.060 | 46.455 | 92.105 |
| Mean | 133.175 | 59.201 | 85.761 |

| **Subject identification code** | **Control product** | | |
|---|---|---|---|
| | **Before use** | **Immediately after use** | **2 weeks after use** |
| 23118-PT1-01 | 136.720 | 51.780 | 100.335 |
| 23118-PT1-02 | 101.030 | 47.565 | 95.655 |
| 23118-PT1-03 | 146.390 | 51.565 | 64.555 |
| 23118-PT1-04 | 128.640 | 56.670 | 127.450 |
| 23118-PT1-05 | 132.095 | 59.260 | 112.140 |
| 23118-PT1-06 | 136.755 | 51.865 | 91.655 |
| 23118-PT1-07 | 150.140 | 52.165 | 102.395 |
| 23118-PT1-08 | 121.315 | 49.090 | 99.395 |
| 23118-PT1-09 | 152.825 | 72.490 | 100.955 |
| 23118-PT1-10 | 134.100 | 53.595 | 108.250 |
| 23118-PT1-11 | 100.790 | 60.375 | 117.305 |
| Mean | 130.982 | 55.129 | 101.826 |

- Test product application site: Compared with before use, statistically significant improvements were observed immediately after use and after 2 weeks of use.
- Control product application site: Compared with before use, statistically significant improvements were observed immediately after use and after 2 weeks of use.
- Between-group comparison: Compared with before use, statistically significant differences between the groups were observed immediately after use and after 2 weeks of use.

### 9. Visual evaluation of skin cracking

### [Test Method]

Visual evaluation of skin cracking was performed with reference to the methodology described in the paper entitled "A new technique for evaluating heel xerosis grade and the effects of moisturizer on heel skin dryness". Two researchers assessed the subjects at three time points: before product use, immediately after use, and after 2 weeks of use.

A lower grade indicates a higher effect in improving cracked heels and the criteria in Table 11 below described in the paper were referenced.

**[Table 11]**

| Scoring | Grade | Description | Standard photograph |
|---|---|---|---|
| 0 | Normal | Normal skin (there are no flakes, scales, fissures or crackles) | 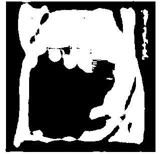 |
| 1 | Mild | Dusty appearance, skin flakes ( < 20%) | 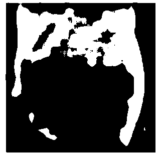 |
| 2 | Moderate | Skin flakes or scales ( > 20%), shallow fissure or cracks | 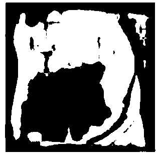 |
| 3 | Severe | Skin flakes or scales ( > 20%), well defined deep and long fissures or cracks | 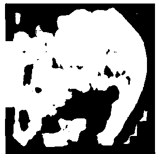 |

### [Test Results]

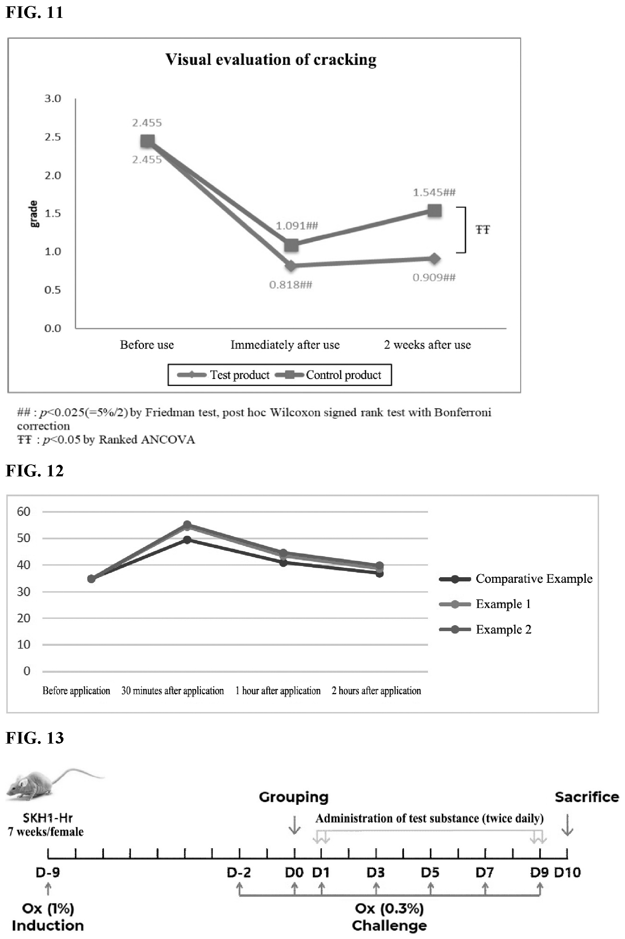

The measurement results of cracking are shown in Table 12 (unit: grade) and FIG. 11.

**[Table 12]**

| **Subject identification code** | **Test product** | | |
|---|---|---|---|
| | **Before use** | **Immediately after use** | **2 weeks after use** |
| 23118-PT1-01 | 3 | 1 | 1 |
| 23118-PT1-02 | 2 | 0 | 1 |
| 23118-PT1-03 | 2 | 2 | 1 |
| 23118-PT1-04 | 2 | 0 | 0 |
| 23118-PT1-05 | 3 | 0 | 2 |
| 23118-PT1-06 | 3 | 2 | 2 |
| 23118-PT1-07 | 2 | 0 | 0 |
| 23118-PT1-08 | 3 | 2 | 1 |
| 23118-PT1-09 | 3 | 1 | 1 |
| 23118-PT1-10 | 2 | 1 | 1 |
| 23118-PT1-11 | 2 | 0 | 0 |
| Mean | 2.455 | 0.818 | 0.909 |

| **Subject identification code** | **Control product** | | |
|---|---|---|---|
| | **Before use** | **Immediately after use** | **2 weeks after use** |
| 23118-PT1-01 | 3 | 1 | 2 |
| 23118-PT1-02 | 2 | 0 | 1 |
| 23118-PT1-03 | 2 | 2 | 1 |
| 23118-PT1-04 | 2 | 0 | 1 |
| 23118-PT1-05 | 3 | 0 | 2 |
| 23118-PT1-06 | 3 | 2 | 2 |
| 23118-PT1-07 | 2 | 1 | 1 |
| 23118-PT1-08 | 3 | 2 | 2 |
| 23118-PT1-09 | 3 | 2 | 2 |
| 23118-PT1-10 | 2 | 1 | 2 |
| 23118-PT1-11 | 2 | 1 | 1 |
| Mean | 2.455 | 1.091 | 1.545 |

- Test product application site: Compared with before use, statistically significant improvements were observed immediately after use and after 2 weeks of use.
- Control product application site: Compared with before use, statistically significant improvements were observed immediately after use and after 2 weeks of use.
- Between-group comparison: Compared with before use, statistically significant differences between the groups were observed immediately after use and after 2 weeks of use.

### 10. Results of Efficacy evaluation survey

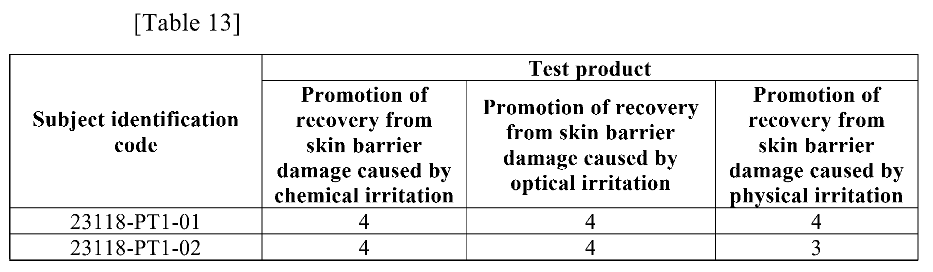

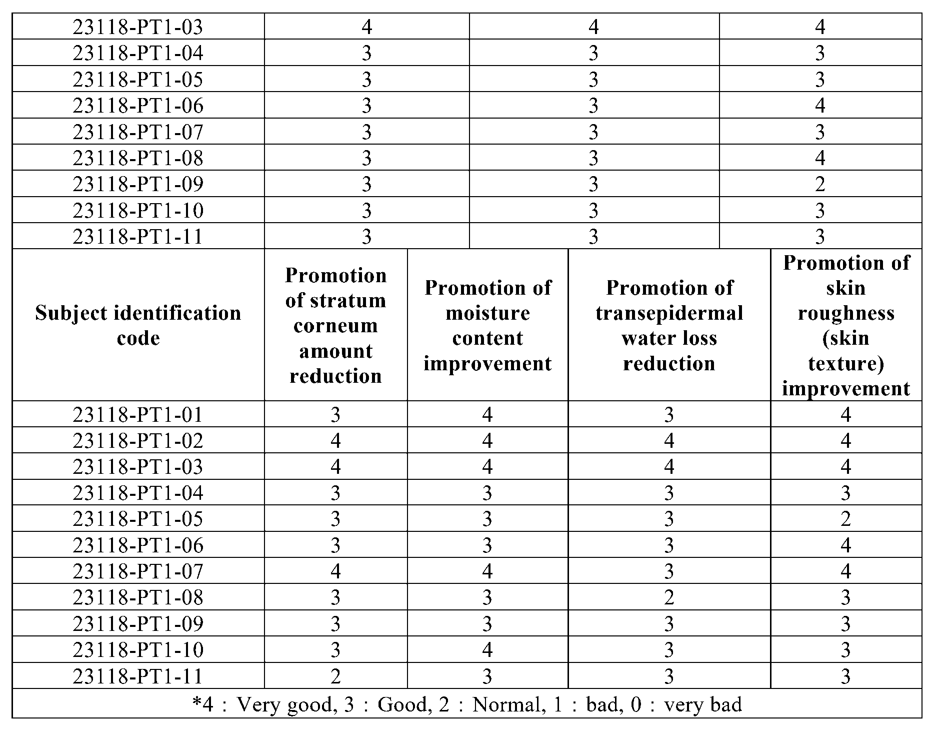

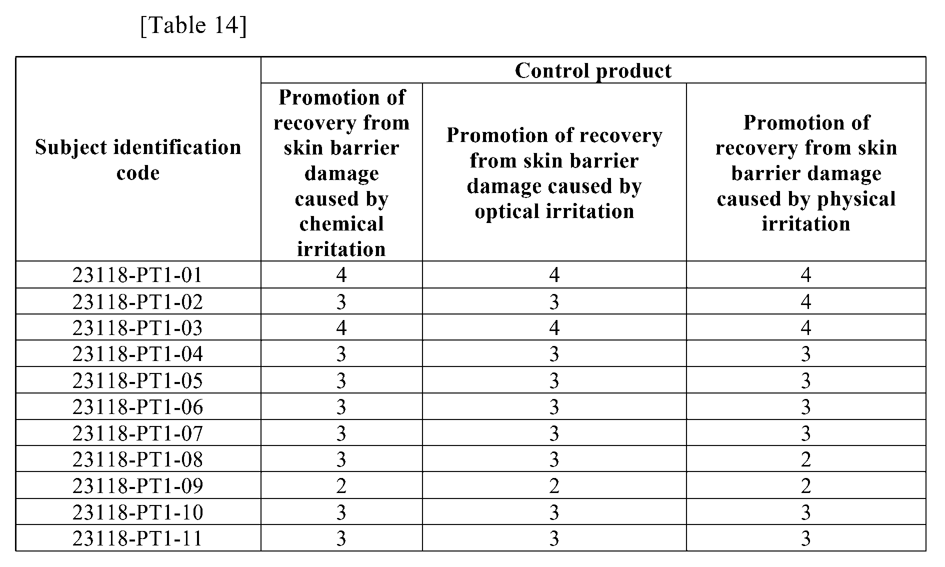

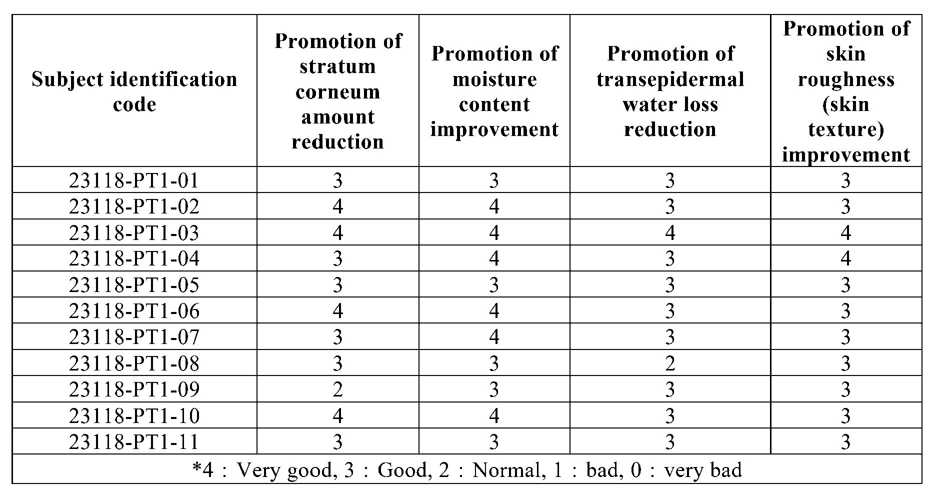

### 11. Results of Product Preference Survey

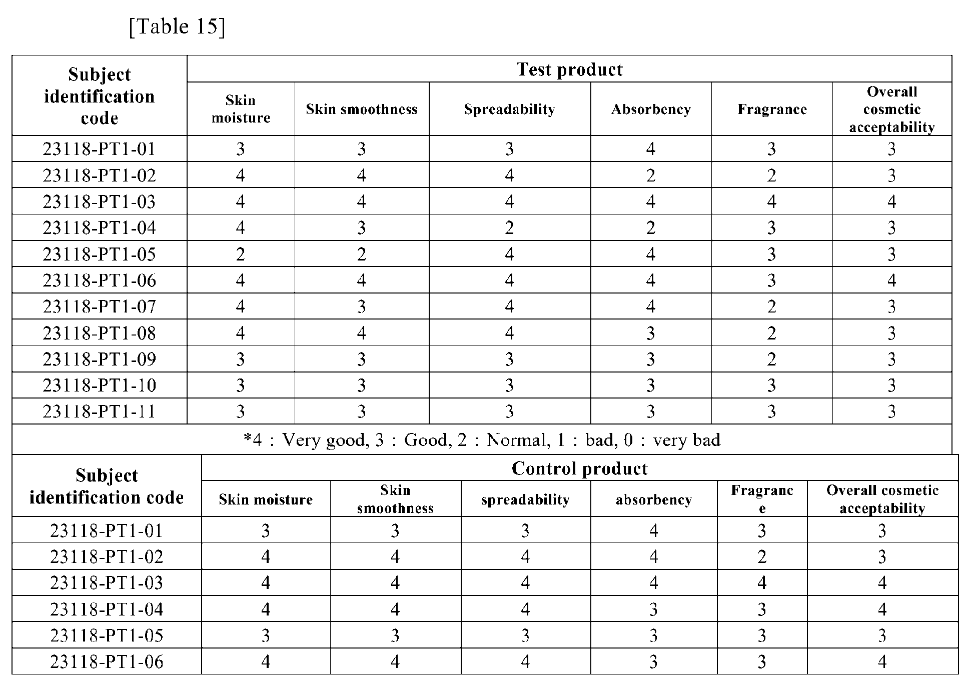

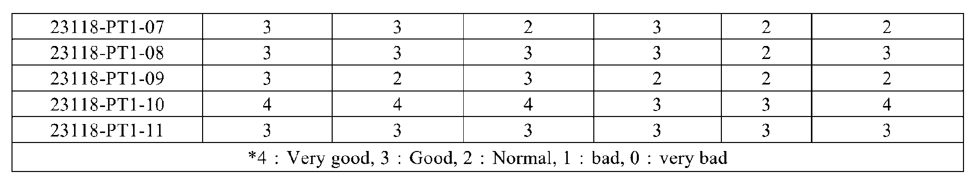

The topical skin composition of the present invention was evaluated for the following effects: recovery from skin barrier damage induced by chemical irritation, recovery from skin barrier damage induced by optical irritation, recovery from skin barrier damage induced by physical irritation, stratum corneum amount, moisture content, transepidermal water loss, skin roughness (texture), and cracking. The results were found to be significant. No specific skin adverse reactions, toxicity, or side effects were observed during the period in which the subjects used the test product.

### Concentration dependency test

To determine concentration dependency, eight researchers applied samples containing Al-BA/(+)BCNF to the forearms of Comparative Example (0%), Example 1 (0.5%), and Example 2 (1.0%). Skin moisture was measured over time using a Corneometer (Cutometer^{®} dual MPA 580). No significant abnormalities were observed.

The results are shown in Table 16 and FIG. 12.

**[Table 16]**

| | Before application | 30 minutes after application | 1 hour after application | 2 hours after application |
|---|---|---|---|---|
| Comparative Example | 34.9 | 49.53 | 40.96 | 36.91 |
| Example 1 | 34.9 | 54.38 | 43.47 | 38.7 |
| Example 2 | 34.9 | 55.19 | 44.58 | 39.83 |

Efficacy evaluation for atopic dermatitis

### 1. Test animals and breeding conditions

### 1.1. Test animals

**[Table 17]**

| Species and strains: | Female hairless mice (CriOri: SKH1-Hrhr) |
|---|---|
| Reasons for selecting test systems: | The mice used in this test were selected because they possess a wealth of accumulated basic test data, making it easy to interpret and evaluate the test results. |
| Source and producer: | SaeronBio Inc. |
| | (251 Anyangpangyo-ro, Uiwang-si, Gyeonggi-do) |
| Date of acquisition: | April 26, 2023 |
| Age at acquisition: | 7 weeks |
| Weight range: | 22g ± 10% |
| Quarantine and acclimatization: | After 7 days of acquisition (during the acclimation period, general symptoms were observed to confirm health and healthy animals were used for test). |
| Subject identification: | Subjects were identified by tail tattoos, and subject identification cards including the management number, population group, animal number, and gender were attached to each breeding room. |
| Number of animals ordered: | 36 |
| Number of animals used: | 36 |

### 1.2. Breeding Conditions

**[Table 18]**

| Breeding Room | Rodent Breeding Area, Room 1, Hospital Digestive Disease Effectiveness Evaluation Support Center, Inha University |
|---|---|
| Ventilation Frequency | 14 to 18 times / hour |
| Temperature and Humidity Range | 22 ± 2°C, 50 ± 10% |
| Lighting Duration and Intensity | 12 hours (lights on 8:00 AM - lights off 8:00 PM), 150 - 300 Lux |
| Breeding boxes | Polysulfonate cage (Tree Shine, Korea)[200 W x 320 D x 145 H(mm)] |
| Number of animals per breeding box | 6 |
| Breeding management | The cage floor was covered with straw to absorb feces and urine, and the cage and straw were replaced twice a week. |
| Type and name of feed | Safe diet [Woojung Bio Co., Ltd.] |
| Feed supply | The animals were placed in a feeder and allowed to take freely. |
| Type of drinking water | Tap water was disinfected using UV sterilizer and microfiltration device. |
| Water supply | The animals were placed in feeder and allowed to take freely. |

### 2. Test materials and methods

### 2.1. Test Substance

AL-BA/BCNF 0.3% Cream: a white cream-type adhesive transparent wound dressing containing 0.3 wt% of Al-BA/(+)BCNF as an active ingredient (solvent is purified water) and prepared by adding a thickener or the like to have a pH of 6.5 to 9 and a viscosity of about 30,000 to 60,000 cP (Brookfield Viscometer, RV type, Spindle No. 6, (10, 15, 20) r/min).

### 2.2. Control Material

### Aestura Atobarrier Itching Cream MD (commercially available) (Manufacturer: Amorepacific)

### Appearance: White cream

### 2.3. Test Method

The overall test group composition and test material types are shown in Table 19 below.

**[Table 19]**

| Test animal | No. of group | Atopic dermatitis induction method | Observation period | Test substance | No. of animals |
|---|---|---|---|---|---|
| Female hairless mice (CriOri:SKH1-Hrhr) | G1 | Induction by application of oxazolone to the skin | 10 days | - | 12 |
| | G2 | | | Adhesive transparent wound dressing (Daebong LS) | 12 |
| | G3 | | | Aestura Atobarrier Itching Cream MD (Amorepacific) | 12 |

Atopic dermatitis induction: Female mice weighing 20-24 g, acclimated for at least 7 days, were subjected to general anesthesia using an inhalant anesthetic (0.5-2% isoflurane: Ifran^{®} Hana Pharm Co Ltd) with oxygen, 1:1 (5-10 ml/kg/min). 1% oxazolone was applied to the skin around the scapula of the anesthetized mice and allowed to absorb. One week later, 0.3% oxazolone was re-administered to induce atopic dermatitis in the absorbed skin. Then, 0.3% oxazolone was administered once every two days to continuously induce atopic dermatitis.

Test substance application: One minute after the third administration of 0.3% oxazolone, the test substance and control cream were applied to the atopic dermatitis-induced area. Administration was repeated twice daily to the same site until the end of the test.

Autopsy: Autopsy was performed 14 days after administration of the test and control substances.

The overall schedule and scheme for the test procedure are shown in FIG. 13.

### 3. Observation and test items

### 3.1. General symptom observation

During the test period, respective animals were observed for clinical symptoms and the occurrence of mortality and moribundity. Records were maintained only for animals exhibiting specific clinical symptoms (normal animals were not recorded).

### 3.2. Atopy severity evaluation

As shown in Table 20 below, four items of erythema/hemorrhage, scaling/dryness, edema, and excoriation/erosion were scored on a scale of 0 to 3 (normal: 0, mild: 1, moderate: 2, severe: 3). The total score was used to assess the severity of atopy.

**[Table 20]**

| | Normal: 0 | Mild: 1 | Moderate: 2 | Severe: 3 |
|---|---|---|---|---|
| Erythema/bleeding | Normal skin color | Slight redness | Redness/microbleeding | Very severe redness/ bleeding |
| Scaling/dryness | Normal | Keratinization of 10% or more of induced area | Keratinization of more than 50% of induced area | Keratinization of 80% or more of induced area |
| Edema | None | 10% or more of induced area | More than 30% of induced area | 50% or more of induced area |
| Excoriation/ erosion | None | 10% or more of induced area | More than 50% of induced area | 80% or more of induced area |

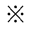 The most severely symptomatic subject was selected and assigned a scale of 3 to establish the evaluation criteria.

### 3.3. Autopsy

Ten days after administration of the test and control substances, the test animals were euthanized using carbon dioxide to terminate the study, and autopsies were performed. For histopathology, skin tissue with induced atopic dematitis were excised and stored in a 10% formalin solution. Only organs with unusual findings were recorded and stored (normal organs were not recorded or stored).

### 3.4. Histopathological examination

Fixed tissues were subjected to routine histological processing, including trimming, dehydration, paraffin embedding, and sectioning, to prepare specimens for histopathological examination. Hematoxylin & Eosin (H&E) staining was performed. The presence of administered substances and histopathological changes were observed using an optical microscope (Olympus BX53, Japan). The specimens were then scanned using a slide scanner (Carl Zeiss Axio Scan Z1, Germany) and the scanned images were analyzed using an image analysis program (Carl Zeiss ZEN, Germany).

### 3.5. Epithelial thickness measurement

The histopathological images scanned using a slide scanner (Carl Zeiss Axio Scan Z1, Germany) were analyzed using ImageJ (Ver. 1.53f51) to measure the epithelial thickness at three points.

### 4. Test Results

### 4.1. Deaths and general symptoms

During the test period, no deaths were observed due to atopic dermatitis or administration of the test or control substances. All subjects with atopic dermatitis had skin erythema/hemorrhage, scaling/dryness, edema, and excoriation/erosion. However, no abnormal symptoms, such as fever or inflammation, were observed due to administration of the test or control substances.

### 4.2. Atopy dermatitis severity

After the test period, all subjects in the atopy dermatitis-induced skin areas was visually inspected and scored for erythema/hemorrhage, scaling/dryness, edema, and excoriation/erosion.

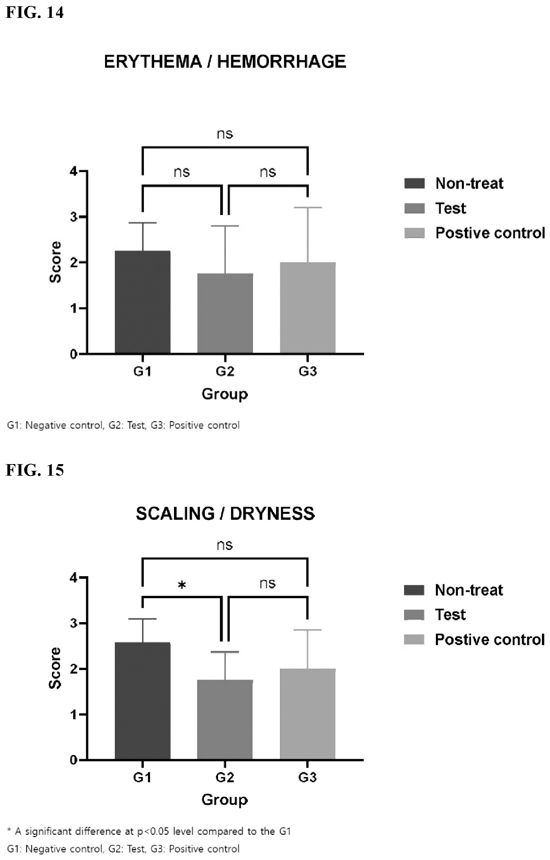

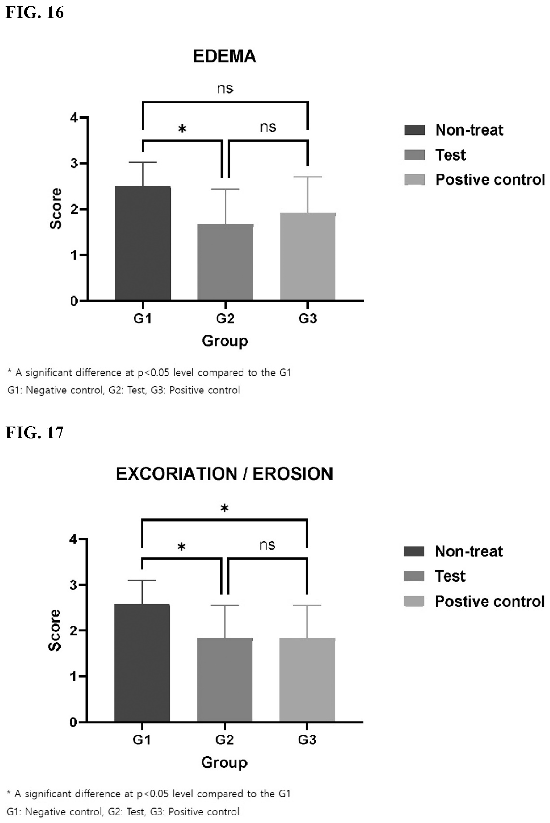

The results are shown in Tables 21 to 24 and FIGS. 14 to 17.

**[Table 21]**

| ERYTHEMA / HEMORRHAGE | | | |
|---|---|---|---|
| Day10 | GROUPS | | |
| | G1 | G2 | G3 |
| GRADE | 2.25 ± 0.62 | 1.75 ± 1.06 | 2.00 ± 1.21 |
| N | 12 | 12 | 12 |

| | | | |
|---|---|---|---|
| G1: Negative control, G2: Test, G3: Positive control Data were expressed as Mean ± S.D. 0 - none 1 - mild 2 - moderate 3 - severe | | | |

**[Table 22]**

| SCALING / DRYNESS | | | |
|---|---|---|---|
| Day10 | GROUPS | | |
| | G1 | G2 | G3 |
| GRADE | 2.58 ± 0.51 | *1.75 ± 0.62 | 2.00 ± 0.85 |
| N | 12 | 12 | 12 |

| | | | |
|---|---|---|---|
| G1: Negative control, G2: Test, G3: Positive control Data were expressed as Mean ± S.D. * A significant difference at p < 0.05 level compared to the G1 0 - none 1 - mild 2 - moderate 3 - severe | | | |

**[Table 23]**

| EDEMA | | | |
|---|---|---|---|
| Day10 | GROUPS | | |
| | G1 | G2 | G3 |
| GRADE | 2.50 ± 0.52 | 1.67 ± 0.78^{*} | 1.92 ± 0.79 |
| N | 12 | 12 | 12 |

| | | | |
|---|---|---|---|
| G1: Negative control, G2: Test, G3: Positive control Data were expressed as Mean ± S.D. * A significant difference at p<0.05 level compared to the G1 0 - none 1 - mild 2 - moderate 3 - severe | | | |

**[Table 24]**

| EXCORIATION / EROSION | | | |
|---|---|---|---|
| Day10 | GROUPS | | |
| | G1 | G2 | G3 |
| GRADE | 2.58 ± 0.51 | 1.83 ± 0.72^{*} | 1.83 ± 0.72^{*} |
| N | 12 | 12 | 12 |

| | | | |
|---|---|---|---|
| G1: Negative control, G2: Test, G3: Positive control Data were expressed as Mean ± S.D. * A significant difference at p<0.05 level compared to the G1 0 - none 1 - mild 2 - moderate 3 - severe | | | |

The mean erythema/hemorrhage scores for the respective groups were 2.25 ± 0.62 in the non-treated group (G1), 1.75 ± 1.06 in the test group (G2), and 2.00 ± 1.21 in the control group (G3). No significant differences were observed between the groups. The mean scores for scaling/dryness in the respective groups were 2.58 ± 0.51 in the non-treated group (G1), 1.75 ± 0.62 in the test group (G2), and 2.00 ± 0.85 in the control group (G3). A significant difference was observed between the non-treated and test groups (G1 vs G2 *p* = 0.0134). No significant difference was observed between the non-treated and control groups. The mean scores for edema in the respective groups were 2.50 ± 0.52 in the non-treated group (G1), 1.67 ± 0.78 in the test group (G2) and 1.92 ± 0.79 in the control group (G3). A significant difference was observed between the non-treated and test groups (G1 vs G2 *p* = 0.0185). No significant difference was observed between the non-treated and control groups. The mean scores for excoriation/erosion in the respective groups were 2.58 ± 0.51 in the non-treated group (G1), 1.83 ± 0.72 in the test group (G2), and 1.83 ± 0.72 in the control group (G3). Significant differences were observed between the non-treated and test groups, and between the non-treated and control groups (G1 vs. G2 *p* = 0.0227, G1 vs. G3 *p* = 0.227). No significant differences were observed between the test and control groups.

### 4.3. Epidermal thickness of atopic dermatitis-induced skin

After the end of the test period, histopathological examinations were performed on the atopic dermatitis-induced skin areas of all subjects. Tissue slides were stained with H&E to measure epidermal thickness and compared between the groups.

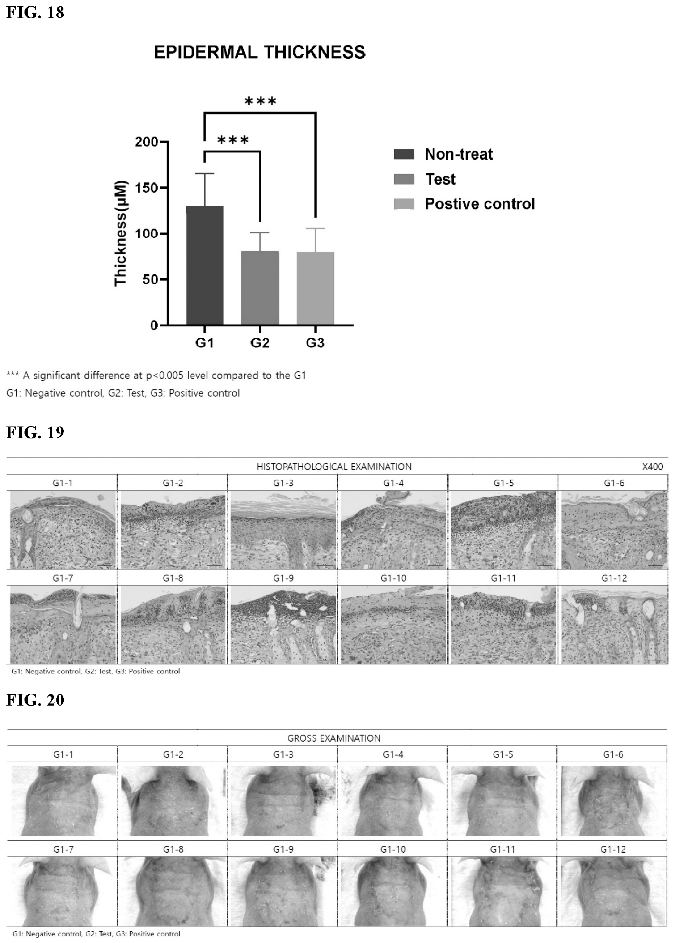

The results are shown in Table 25 and FIG. 18.

**[Table 25]**

| EPIDERMAL THICKNESS (*µ*m) | | | |
|---|---|---|---|
| Day10 | GROUPS | | |
| | G1 | G2 | G3 |
| GRADE | 129.42 ± 36.27 | 80.48 ± 20.91^{***} | 79.55 ± 26.20^{***} |
| N | 12 | 12 | 12 |

| | | | |
|---|---|---|---|
| G1: Negative control, G2: Test, G3: Positive control Data were expressed as Mean ± S.D. *** A significant difference at p<0.005 level compared to the G1 | | | |

The thickness of the epidermal skin for the respective groups was 129.42 ± 36.27 µm in the non-treated group (G1), 80.48 ± 20.91 µm in the test group (G2), and 79.55 ± 26.20 µm in the control group (G3). It was found that there was a significant difference between the test group and the control group and the non-treated group (G1 vs G2 *p* = 0.0005, G1 vs G3 *p* = 0.0004). No significant difference was observed between the test group and the control group.

### 7. Discussion and conclusion

For induction of the atopic dermatitis model and application of the test substance, 1% oxazolone was administered to the skin around the scapula of female hairless mice (CriOri:SKH1-Hrhr) acclimated for at least 5 days. 0.3% oxazolone was re-administered to the mice one week later to induce atopic dermatitis. Subsequently, 0.3% oxazolone was administered once every two days to induce sustained atopic dermatitis. One minute after the third 0.3% oxazolone administration, the test substance and control cream were applied to the atopic dermatitis-induced area. The administeration was performed on the same area twice daily until the end of the test. Autopsies were performed 10 days after administration of test and control substances.

The groups were divided into three groups: G1: non-treated group (Negative Control, Non-treated), G2: test group (Test: AL-BA/BCNF 0.3% cream application), and G3: control group (Positive Control: Estura Atobarrier Itching Cream Application). 36 mice were used in total, with 12 mice in each group.

During the test period, no deaths or treatment-related clinical signs attributable to induction of atopic dermatitis or administration of the test or control substance were observed.

After the test period, all mice were visually evaluated for erythema/hemorrhage, scaling/dryness, edema, and excoriation/erosion at the atopic dermatitis and test substance administration sites. No significant differences in erythema/hemorrhage were observed between groups. Significant differences were observed in scaling/dryness and edema symptoms between the non-treated and test groups, but no significant differences were observed between the non-treated and control groups. Significant differences were observed in excoriation/erosion symptoms between the non-treated and treated groups (test and control groups), but no significant differences were observed between the test and control groups.

Significant differences in epidermal thickness were observed between the non-treated group and the test group, and between the non-treated group and the control group, but no significant differences were observed between the test group and the control group.

The test results showed that the test and control substances significantly alleviated the symptoms of atopic dermatitis and significantly restored epidermal thickness damaged by atopic dermatitis. However, no significant differences were observed between the test and control substances. No clinical indicators indicating toxicity caused by administration of the test and control substances were observed.

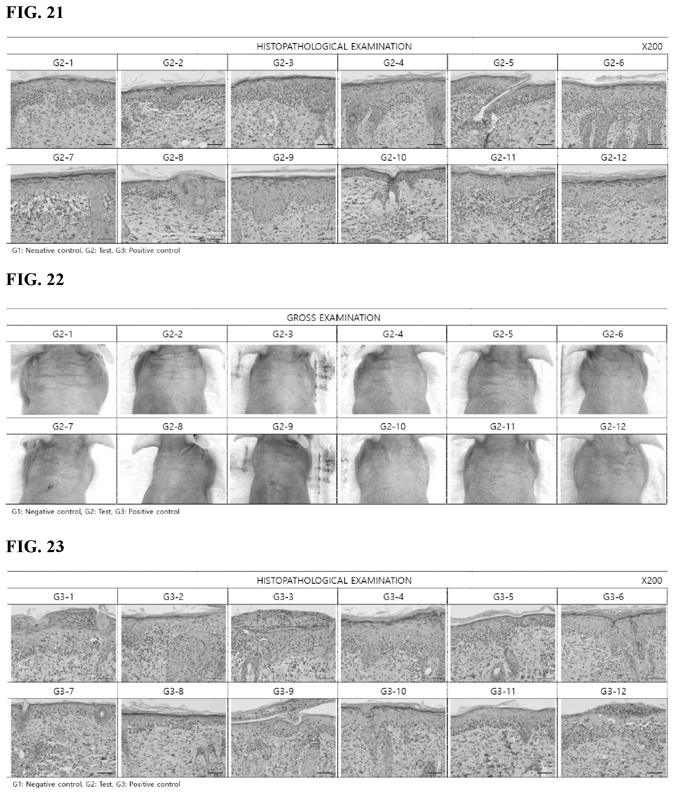

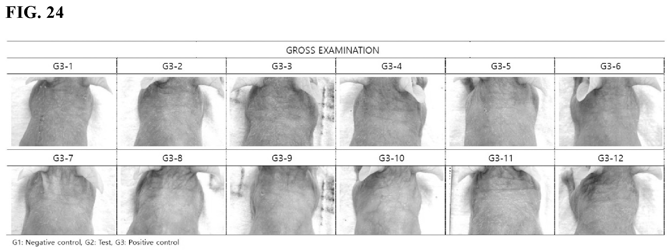

Meanwhile, the results of gross and histopathological examinations are shown in FIGS. 19 to 24.

The present invention showed that the test substance, AL-BA/BCNF 0.3% cream adhesive transparent wound dressing, was non-inferior to the reference substance, Aestura Atobarrier Itching Cream, in terms of safety and efficacy in atopic dermatitis.

### [Industrial Applicability]

The present invention may be widely used in the fields of health products, pharmaceuticals, medical devices, cosmetics, or the like.

## Claims

1. A skin topical composition, as active ingredients, comprising:
(a) an Al-BA compound, in which phenylboronic acid is conjugated to alginate; and
(b) bacterial cellulose nanofibers having a positively charged surface ((+)BCNF).

2. The skin topical composition according to claim 1, wherein the composite of the Al-BA compound and (+)BCNF is present in an amount of 0.1 to 10 wt% based on a total weight of the composition.

3. The skin topical composition according to claim 1, wherein the Al-BA compound and the bacterial cellulose nanofibers (BCNF) are present in a weight ratio of 1:0.01 to 1:0.5.

4. The skin topical composition according to claim 1, further comprising one or more moisturizing agents or skin barrier ingredients selected from the group consisting of glycerin, hyaluronic acid, and cholesterol.

5. The skin topical composition according to any one of claims 1 to 4, wherein the composition is a cosmetic composition for restoring skin barrier function and enhancing skin moisturization.

6. The skin topical composition according to any one of claims 1 to 4, wherein the composition is a quasi-drug composition for protecting skin in areas where the skin barrier is damaged.

7. The skin topical composition according to claim 6, wherein the quasi-drug composition is provided as an adhesive wound dressing.

8. The skin topical composition according to any one of claims 1 to 4, wherein the composition is a pharmaceutical composition for treating, preventing, or alleviating one or more diseases or symptoms selected from the group consisting of xerosis, pruritus, asteatotic eczema, cutaneous hyperkeratosis, keratinization of the heel and malleolar regions, senile xerosis, rough skin in adults, photodermatitis, atopic dermatitis, and psoriasis.
